# EUROPÄISCHE PATENTSCHRIFT

(11) **EP 0 862 377 B1**
(45) Veröffentlichungstag und Bekanntmachung des Hinweises auf die Patenterteilung: **24.08.2005**
(21) Anmeldenummer: 96938160.7
(22) Anmeldetag: 08.11.1996
(51) Int. Cl.: A61B 1/12

(54) **VERFAHREN UND VORRICHTUNG ZUM SPÜLEN**
METHOD AND DEVICE FOR RINSING
PROCEDE ET DISPOSITIF DE RINCAGE

(30) Priorität: 10.11.1995 DE 19542020; 06.12.1995 DE 19545528
(43) Veröffentlichungstag der Anmeldung: 09.09.1998
(73) Patentinhaber: Erbe Elektromedizin GmbH, 72072 Tübingen (DE)
(72) Erfinder: FARIN, Günter, D-72070 Tübingen (DE); FISCHER, Klaus, D-72202 Nagold (DE); GRUND, Karl, Ernst, D-72070 Tübingen (DE); WURSTER, Helmut, D-75038 Oberderdingen (DE)
(74) Vertreter: Bohnenberger, Johannes
(86) Internationale Anmeldenummer: PCT/EP1996/004905
(87) Internationale Veröffentlichungsnummer: WO 1997/017010

(56) Entgegenhaltungen:
- EP-A- 0 051 862
- EP-A- 0 340 145
- EP-A- 0 437 229
- PATENT ABSTRACTS OF JAPAN vol. 013, no. 286 (C-613), 29.Juni 1989 & JP 01 083237 A (FUJI PHOTO OPTICAL CO LTD), 29.März 1989,

## Beschreibung

Die Erfindung betrifft ein Verfahren und eine Vorrichtung zum Spülen eines Behandlungsbereiches oder Operationsfeldes beim Endoskopieren nach dem Oberbegriff des Patentanspruches 1 bzw. des Patentanspruches 2.

Beim Endoskopieren, also bei der Untersuchung von Körperhöhlen mittels eines Endoskops sowie bei chirurgischen Eingriffen mittels eines Endoskops geschieht es oftmals, daß das Betrachtungs- oder Operationsfeld von Verschmutzungen befreit werden muß, um ein freies Sichtfeld zu haben bzw. den eigentlichen Operationsbereich zu säubern. Besonders dann, wenn das zu entfernende Material sehr fest ist bzw. fest an der Wand der Körperhöhle sitzt, ist das Freilegen der interessierenden Bereiche schwierig.

Zum Spülen bzw. Reinigen des Behandlungsbereiches bzw. Operationsfeldes sind Spüleinrichtungen bekannt, mit welchen eine Spülflüssigkeit mit einer einstellbaren Strömungsrate durch einen Spül- oder Instrumentierkanal eines Endoskops auf den zu spülenden oder zu reinigenden Behandlungsbereich bzw. das Operationsfeld appliziert werden kann. Diese Spüleinrichtungen haben jedoch den Nachteil, daß zum Entfernen fester bzw. fest an der Organwand haftender Materialien sehr viel Spülflüssigkeit in die betreffende Körperhöhle bzw. in das betreffende Hohlorgan eingebracht wird.

Aus der DE 40 00 410 A1 ist ein Endoskop bekannt, das eine Spüleinrichtung aufweist, welche die Applikation eines Luft-Flüssigkeits-Gemisches durch einen Instrumentierkanal des Endoskops ermöglicht. Hierfür weist diese Spüleinrichtung ein Pumpenaggregat auf, welches sowohl mit einer Wasserpumpe als auch mit einer Luftpumpe ausgestattet ist, die gleichzeitig oder alternierend, automatisch oder schaltgesteuert aktiviert werden können, so daß durch den Instrumentierkanal hindurch ein Luft-Flüssigkeits-Gemisch in den Behandlungsbereich bzw. auf das Operationsfeld zugeführt wird. Das Luft-Flüssigkeits-Gemisch wird im Pumpenaggregat in einem Verbindungsstück zusammengeführt und von dort aus durch eine einzige, gemeinsame Sammelleitung zu einem Abzweig am proximalen Endes des Instrumentierkanals geführt, und zwar auch dann, wenn an das Verbindungsstück sowohl eine Wasserpumpe als auch eine Luftpumpe angeschlossen sind.

Diese Spüleinrichtung hat im Vergleich zu den oben genannten Spüleinrichtungen zwar den Vortiel, daß weniger Wasser in die Körperhöhle bzw. in das zu behandelnde Organ eingebracht wird, die Effektivität bezüglich der Entfernung fester bzw. fest an der Organwand haftender Materialien ist jedoch noch zu gering. Eine Erhöhung der Effektivität durch Erhöhung des Drucks des Luft-Flüssigkeits-Gemisches wird durch die begrenzte Druckfestigkeit der Instrumentierkanäle flexibler Endoskope limitiert.

Der Erfindung liegt die Aufgabe zugrunde, auf einfache Weise bei verringerter Flüssigkeitsmenge und möglichst geringem Druck eine verbesserte Reinigungswirkung zu erzielen.

Diese Aufgabe wird durch die im Patentanspruch 1 angegebenen Merkmale gelöst.

Ein wesentlicher Punkt der Erfindung liegt darin, daß die Reinigungsflüssigkeit nicht wie bisher üblich in einem im wesentlichen kontinuierlichen Strahl appliziert sondern in Form von separaten Paketen, sozusagen in Form von "Geschossen" auf die zu entfernenden "Verunreinigungen" gerichtet wird, wobei die Energie eines solchen "Geschosses" in erhöhtem-Maße durch seine Geschwindigkeit bestimmt wird und diese Geschwindigkeit wiederum durch den Gasdruck und die (besonders bei sehr langen Endoskopen in erhöhtem Maße zur Verfügung stehende) Laufstrecke des einzelnen geschoßartig wirkenden Flüssigkeitspfropfens in weiten Bereichen einstellbar ist. Die dabei eingebrachte Flüssigkeitsmenge ist entsprechend gering und darüber hinaus deshalb sehr leicht einstellbar, weil man mit einzelnen (geringen) Flüssigkeitsmengen arbeitet. Ein weiterer Vorteil liegt noch darin, daß mit einem relativ niedrigen Druck (dieser könnte für den Patienten gefährlich sein und die Druckfestigkeit des Arbeitskanals eines Endoskops übersteigen) gearbeitet werden kann, da die Energie der einzelnen Flüssigkeitspfropfen über die (lange) Laufstrecke gesteigert wird, bis sie an der Austrittsöffnung (evtl. des Endoskopkanals) ihren Maximalwert im wesentlichen erreicht haben.

Vorzugsweise wird eine Vielzahl von Flüssigkeitstropfen nacheinander, insbesondere in regelmäßigen Abständen ausgestoßen, wobei die Pausen zwischen den Tropfen nicht allzu kurz sein sollten, um mit minimalen Flüssigkeitsmengen arbeiten und den Reinigungsvorgang sofort dann abbrechen zu können, wenn das gewünschte Ziel erreicht wurde. Vorzugsweise sind hierbei sowohl die Flüssigkeitsmenge eines jeden Flüssigkeitspfropfens (Geschosses), der Druck des die Flüssigkeitspfropfen beschleunigenden Gases sowie die Abstände, in denen die Flüssigkeitspfropfen die Mündung der Leitung verlassen, einstellbar.

Bei einer bevorzugten Ausführungsform der Erfindung sind steuerbare Ventile vorgesehen, über welche die Leitung abwechselnd mit einer Flüssigkeits- und einer Druckgasquelle verbindbar ist. Dadurch ist eine optimale Steuerbarkeit erzielt. Bei einer besonders einfachen Ausführungsform der Erfindung ist der proximale Abschnitt der Leitung über ein nur in Richtung auf die Mündung der Leitung durchströmbares Ventil mit einer Flüssigkeitsquelle verbunden, die unter einem ersten Druck steht und außerdem mit einer Druckgasquelle, deren Druck zeitlich alternierend den ersten Druck über- und unterschreitet. Dadurch ist gewährleistet, daß immer dann, wenn der Gasdruck den Flüssigkeitsdruck unterschreitet, der Flüssigkeitspfropfen in die Leitung eingeführt und bei einem erneuten Ansteigen des Gasdrucks über den Flüssigkeitsdruck der Flüssigkeitspfropfen beschleunigt und ausgestoßen wird.

Bevorzugte Ausführungsformen der Erfindung ergeben sich aus den Unteransprüchen und der nachfolgenden Beschreibung von Ausführungsbeispielen, die anhand von Abbildungen näher erläutert werden.

Hierbei zeigen:
- Fig. 1: eine schematisierte Darstellung der Erfindung mit zwei Ventilen,
- Fig. 2: ein Zeitdiagramm zur Erläuterung der Wirkungsweise der Anordnung bzw. Steuerung der Ventile,
- Fig. 3: eine weitere Ausführungsform der Erfindung mit einem 3-Wege-Ventil,
- Fig. 4: eine weitere Ausführungsform der Erfindung mit pulsierender Druckgasquelle und
- Fig. 5: ein Zeitdiagramm zur Erläuterung der Wirkungsweise der Anordnung nach Fig. 4.

Bei der nachfolgenden Beschreibung werden gleiche oder gleichwirkende Teile mit denselben Bezugsziffern versehen.

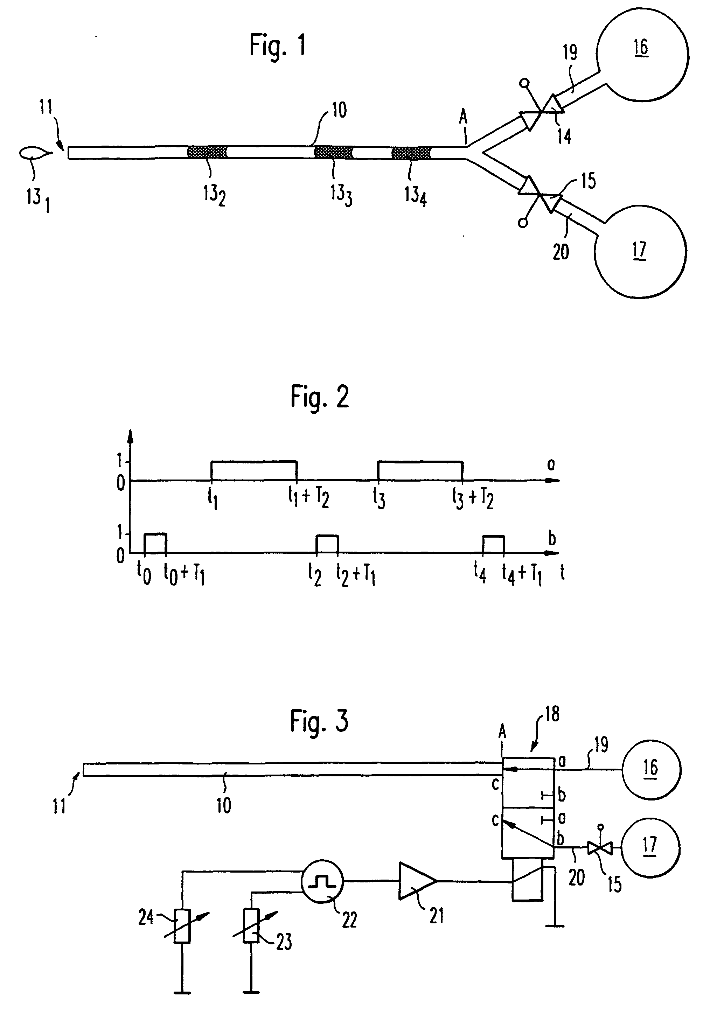

Die in Fig. 1 stark schematisiert gezeigte Anordnung umfaßt eine Leitung 10, welche der Arbeitskanal eines Endoskops oder ein in dieses eingeführter Schlauch oder ein Rohr sein kann. Die Leitung 10 steht über eine erste Verbindungsleitung 19 mit einer Druckgasquelle 16 (eine Pumpe oder Gasflasche oder Gasanschluß) und über eine zweite Verbindungsleitung 20 mit einer Flüssigkeitsquelle 17 in Verbindung. Der Verbindungspunkt ist mit "A" bezeichnet und liegt so nahe am Endoskop bzw. an der Mündung 11 der Leitung 10, daß die im wesentlichen maximale und weiter unten erläuterte "Mündungsgeschwindigkeit" erreicht wird. Die Ausgänge der Ventile (insbesondere des Gas-Ventils 14) liegen möglichst nahe beim Verbindungspunkt A, insbesondere am proximalen Ende des Endoskop-Arbeitskanals. Die erste Verbindungsleitung 19 ist über ein erstes steuerbares Ventil 14, die zweite Verbindungsleitung 20 über ein zweites steuerbares Ventil 15 absperrbar. Die Funktionsweise wird im folgenden beschrieben, wobei die geöffnete bzw. die geschlossene Stellung der Ventile 14, 15 mit "1" für geöffnet und "0" für geschlossen in Fig. 2 dargestellt ist. Der obere mit a bezeichnete Zeitverlauf erläutert die Ansteuerung des Ventils 14, der untere mit b bezeichnete Zeitverlauf erläutert die Ansteuerung des Ventils 15.

Zu einem ersten Zeitpunkt t₀ öffnet das zweite Ventil 15 und bleibt für einen definierten Zeitraum T₁ offen, schließt also zum Zeitpunkt t₀ + T₁ wieder. Während dieser Zeitdauer wird eine bestimmte Flüssigkeitsmenge aus der Flüssigkeitsquelle 17 in die Leitung 10 eingeführt und bildet dort einen "Pfropfen" 13.

Zu einem Zeitpunkt t₁, der nach dem Zeitpunkt t₀ + T₁ liegt, wird das erste Ventil 14 geöffnet, so daß die Druckgasquelle 16 mit der Leitung 10 in Verbindung steht. Die Öffnungsdauer ist in Fig. 2 mit T₂ bezeichnet. Durch das unter Druck stehende Gas wird der Flüssigkeitspfropfen 13 beschleunigt und bewegt sich in Richtung auf die Mündung 11 der Leitung 10. Nach dem Schließen des Ventils 14 beginnt der beschriebene Vorgang zu einem Zeitpunkt t₂ von neuem, zu welchem das Ventil 15 für den vorbestimmten Zeitraum T₁ öffnet. Es werden also Flüssigkeitspfropfen 13₁, 13₂, 13₃ und 13₄ nacheinander in die Leitung 10 eingeführt und durch die Druckgasquelle 16 beschleunigt, so daß sie geschoßartig aus der Mündung 11 austreten und auf die zu reinigende Fläche (nicht gezeigt) aufprallen. Das Gaspolster, das den jeweiligen Flüssigkeitspfropfen beschleunigt, soll zu diesem Zeitpunkt (Austritt aus der Mündung) im wesentlichen den Umgebungsdruck, der Flüssigkeitspfropfen soll seine maximale Geschwindigkeit (Energie) erreicht haben. -

Die in Fig. 3 gezeigte Ausführungsform der Erfindung unterscheidet sich von der nach Fig. 1 dadurch, daß nicht zwei gesonderte Ventile 14 und 15 sondern ein 3-Wege-Ventil 18 vorgesehen ist, dessen zwei Eingangsanschlüsse a und b über die Leitungen 19 und 20 mit der Druckgasquelle 16 bzw. der Flüssigkeitsquelle 17 und dessen Ausgangsanschluß c mit der Leitung 10 verbunden sind. Die Ansteuerung des 3-Wege-Ventils 18 geschieht über einen Verstärker 21, dessen Eingang mit dem Ausgang einer Steuerung 22 verbunden ist, welche das Ventil 18 mit einer über ein erstes Einstellorgan 23 vorgegebenen Frequenz und mit einer Pulsdauer ansteuert, die über ein zweites Einstellorgan 24 einstellbar ist. Weiterhin ist in der zweiten Verbindungsleitung 20 wieder ein zweites Ventil 15 vorgesehen, das bei Betrieb der Anordnung geöffnet und beim Anhalten des Betriebs geschlossen ist, wobei dann das 3-Wege-Ventil 18 in der Stellung b - c angehalten wird. In der Funktion unterscheidet sich die Anordnung nach Fig. 3 von dem in Fig. 2 gezeigten Diagramm dadurch, daß zwischen den Zeitpunkten t₀ + T₁ und t₁ sowie t₁ + T₂ und t₂ (usw.) nur sehr geringe Pausen sind, welche durch die Umsteuerzeiten des 3-Wege-Ventils 18 definiert sind.

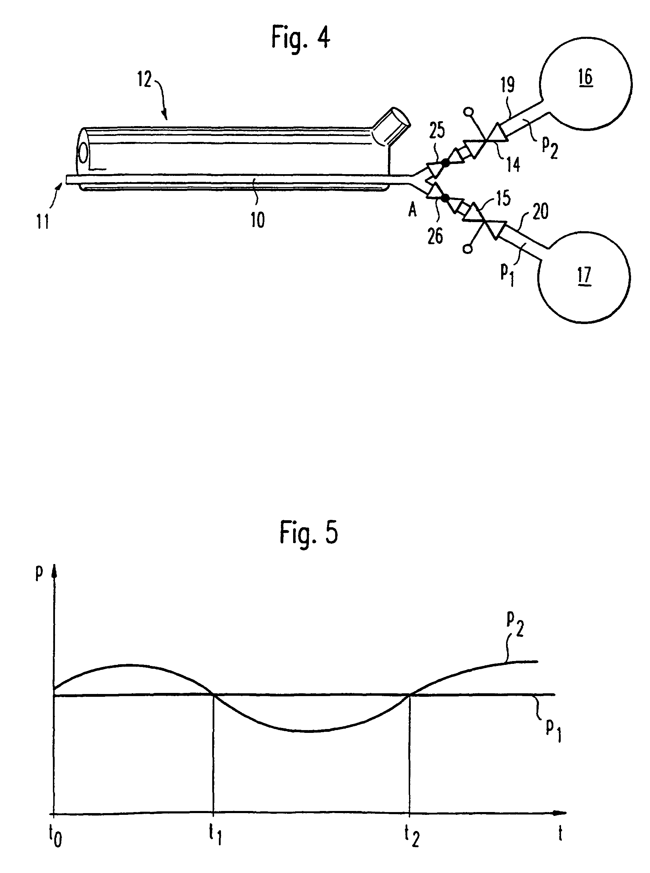

In Fig. 4 ist eine weitere Ausführungsform der Erfindung schematisch dargestellt. Diese umfaßt wieder eine Leitung 10, welche durch den Arbeitskanal eines Endoskops 12 so hindurchgeschoben ist, daß ihre Mündung 11 aus dem Endoskop herausragt.

Am proximalen Ende der Leitung 10 sind über die Verbindungsleitungen 19 und 20 wieder eine Druckgasquelle 16 und eine (Druck-) Flüssigkeitsquelle 17 angeschlossen. In den Leitungen 19 und 20 sind neben den schon oben beschriebenen Ventilen 14 und 15 ein erstes Rückschlagventil 25 und ein zweites Rückschlagventil 26 vorgesehen. Die Druckgasquelle 16 liefert Gas mit einem ersten Druck p₂, die Flüssigkeitsquelle 17 liefert die Flüssigkeit mit einem zweiten Druck p₁. Der Verlauf des Ausgangsdrucks p₁ bzw. p₂ ist in Fig. 5 dargestellt.

Während nun der Druck p₂ der Druckgasquelle 16 oszilliert, also über die Zeit t ansteigt und wieder absinkt, ist der Druck p₁ der Flüssigkeitsquelle 17 im wesentlichen konstant. Der Druck p₂ schwankt nun um den Druck p₁ derart, daß er (in Fig. 5) im Zeitraum t₀ bis t₁ oberhalb des Drucks p₁ liegt und im Zeitraum t₁ bis t₂ unterhalb des Drucks p₁ liegt. Nach t₂ steigt der Druck p₂ wieder über den Druck p₁ an. Durch diese Druckverhältnisse ist gewährleistet, daß im Zeitraum t₁ bis t₂ Spülflüssigkeit aus dem Spülflüssigkeitsbehälter 29 in die Leitung 10 gelangt und im Zeitraum t₀ bis t₁ bzw. nach t₂ der so in die Leitung 10 eingefüllte Flüssigkeitspfropfen durch Gas aus der Druckgasquelle 16 die Leitung 10 entlang beschleunigt und aus der Mündung 11 ausgestoßen wird. Die Ventile 14 und 15 dienen also hier lediglich zum An- und Abschalten des Prozesses, während die Steuerung durch die Oszillation der Druckgasquelle vorgenommen wird. Wichtig ist auch bei dieser Anordnung, daß die Leitungslängen derart gehalten sind, daß die einzelnen Flüssigkeitspfropfen wie oben erläutert (entsprechend den eingestellten Druck- und Mengenverhältnissen) mit maximaler Energie aus der Mündung 11 ausgestoßen werden.

Selbstverständlich ist es auch möglich, den Druck p₁ der Spülflüssigkeit nicht konstant zu halten, sondern gegenphasig zum Druck p₂ verlaufen zu lassen bzw. p₂ konstant zu halten und p₁ oszillieren zu lassen.

Ein weiterer Vorteil der Erfindung liegt darin, daß man Gas oder Flüssigkeit auch jeweils allein verwenden kann, ohne ein gesondertes Gerät zu benötigen. Dadurch, daß die Ventile nahe am Verbindungspunkt A liegen, wird beim Umschalten von Gas auf Flüssigkeit (und umgekehrt) nur eine minimale Restmenge des jeweils anderen Mediums ausgestoßen. Die Strömungsraten und Drücke der beiden Medien werden vorzugsweise in recht weiten Grenzen einstellbar gemacht, so daß man eine maximale Spül- bzw. Reinigungsvariabilität mit einem einzigen Gerät erreicht.

### Bezugszeichenliste

- 10: Leitung
- 11: Mündung
- 12: Endoskop
- 13: Flüssigkeitspfropfen
- 14: erstes Ventil
- 15: zweites Ventil
- 16: Druckgasquelle
- 17: Flüssigkeitsquelle
- 18: 3-Wege-Ventil
- 19: erste Verbindungsleitung
- 20: zweite Verbindungsleitung
- 21: Verstärker
- 22: Steuerung
- 23: erstes Einstellorgan
- 24: zweites Einstellorgan
- 25: 1. Rückschlagventil
- 26: 2. Rückschlagventil

## Patentansprüche

1. Vorrichtung zum Spülen eines Beobachtungsbereiches oder Operationsfeldes beim Endoskopieren mit einer Leitung (10) zum Zuführen eines Spülmediums zu einem distalen Bereich eines Endoskops (12) und mit Flüssigkeitszufuhreinrichtungen (14-29) zum Zuführen des Spülmediums in die Leitung (10), wobei das Spülmedium eine Flüssigkeit und ein Gas umfasst,
**dadurch gekennzeichnet,dass**
die Flüssigkeitszufuhreinrichtungen (14-29) derart ausgebildet sind, dass eine einen Flüssigkeitspfropfen (13) definierende Flüssigkeitsmenge in einen proximalen Abschnitt der Leitung (10) gefüllt und dieser Flüssigkeitspfropfen (13) mittels des Gases, das unter Druck nach der Flüssigkeitsmenge in die Leitung (10) eingeführt wird, in der Leitung (10) beschleunigt und mit erhöhter End- oder Mündungsgeschwindigkeit aus einer Mündung (11) der Leitung (10) ausgestoßen wird, wobei die Flüssigkeitszufuhreinrichtungen (14-29) weiterhin erste Einstelleinrichtungen (23) aufweisen, zum Einstellen der Flüssigkeitsmenge eines jeden Flüssigkeitspfropfen (13).

2. Vorrichtung nach Anspruch 1,
**dadurch gekennzeichnet, daß**
die Flüssigkeitszufuhreinrichtungen (14-29) derart ausgestaltet sind, daß eine Vielzahl von Flüssigkeitspfropfen (13) nacheinander, vorzugsweise in regelmäßigen Abständen ausgestoßen wird.

3. Vorrichtung nach einem der Ansprüche 1 bis 2,
**dadurch gekennzeichnet, daß**
die Flüssigkeitszufuhreinrichtungen (14-29) zweite Einstelleinrichtungen zum Einstellen des Druckes (p₁) des Gases umfassen.

4. Vorrichtung nach Anspruch 2,
**dadurch gekennzeichnet, daß**
die Flüssigkeitszufuhreinrichtungen (14-29) dritte Einstelleinrichtungen (24) umfassen, zum Einstellen der Abstände der Flüssigkeitspfropfen (13), mit welchen diese ausgestoßen werden.

5. Vorrichtung nach einem der Ansprüche 1 bis 4,
**dadurch gekennzeichnet, daß**
die Flüssigkeitszufuhreinrichtungen (14-29) steuerbare Ventile (18-20) umfassen, über welche die Leitung (10) abwechselnd mit einer Flüssigkeitsquelle (17) und einer Druckgasquelle (16) verbindbar ist.

6. Vorrichtung nach einem der Ansprüche 1 bis 5,
**dadurch gekennzeichnet, daß**
der proximale Abschnitt der Leitung (10) über ein nur in Richtung auf die Mündung (11) durchströmbares (Rück schlag-) Ventil (26) mit einer Flüssigkeitsquelle (17) verbunden ist, die unter einem ersten Druck (p₁) steht und außerdem mit einer Druckgasquelle (16) verbunden ist, deren Druck (p₂) zeitlich alternierend den ersten Druck (p₁) über- und unterschreitet.

## Claims

1. An apparatus for flushing an observation area or operating zone when using an endoscope, with a conduit (10) for feeding a flushing medium to a distal zone of an endoscope (12) and with liquid-supply means (14-29) for feeding the flushing medium into the conduit (10), wherein the flushing medium comprises a liquid and a gas,
**characterised in that**
the liquid-supply means (14-29) are designed so that a quantity of liquid defining a drop of liquid (13) is charged into a proximal portion of the conduit (10) and this drop of liquid (13) is accelerated in the conduit (10) by means of the gas, which is admitted into the duct (1) under pressure after the quantity of liquid, and is expelled at elevated final or muzzle velocity from an orifice (11) of the conduit (10), wherein the liquid-supply means (14-29) also have first adjustment means (23) for adjusting the quantity of liquid of each drop of liquid (13).

2. An apparatus according to Claim 1, **characterised in that** the liquid-supply means (14-29) are designed so that a plurality of drops of liquid (13) are expelled successively, preferably at regular intervals.

3. An apparatus according to either one of Claims 1 and 2, **characterised in that** the liquid-supply means (14-29) comprise second adjusting means for adjusting the pressure (p₁) of the gas.

4. An apparatus according to Claim 2, **characterised in that** the liquid-supply means (14-29) comprise third adjusting means (24) for adjusting the intervals at which the drops of liquid (13) are expelled.

5. An apparatus according to any one of Claims 1 to 4, **characterised in that** the liquid-supply means (14-29) comprise controllable valves (18-20) by way of which the conduit (1) can be alternately connected to a liquid source (17) and a compressed-gas source (16).

6. An apparatus according to any one of Claims 1 to 5, **characterised in that** the proximal portion of the conduit (10) is connected by way of a (non-return) valve (26), through which the flow can pass only in the direction of the orifice (11), to a liquid source (17), which is at a first pressure (p₁), and additionally to a compressed-gas source (16), the pressure (p₂) of which alternating in time exceeds and falls below the first pressure (p₁).

## Revendications

1. Dispositif de rinçage d'une zone d'observation ou d'un champ d'opération lors d'une endoscopie, comportant une conduite (10), destinée à amener un milieu de rinçage vers une zone distale d'un endoscope (12), et des dispositifs d'amenée de fluide (14-29) destinés à amener le milieu de rinçage dans la conduite (10), le milieu de rinçage comportant un fluide et un gaz,
**caractérisé en ce que** les dispositifs d'amenée de fluide (14-29) sont conçus de façon telle qu'une quantité de fluide définissant un tampon de fluide (13) est remplie dans un segment proximal de la conduite (10) et que ce tampon de fluide (13) est accéléré dans la conduite (10) au moyen du gaz qui est introduit sous pression dans la conduite (10) après la quantité de fluide, et est expulsé hors d'une embouchure (11) de la conduite (10) avec une vitesse finale, ou à l'embouchure, augmentée, les dispositifs d'amenée de fluide (14-29) présentant de plus des premiers dispositifs de réglage (23) destinés à régler la quantité de fluide d'un tampon de fluide (13) respectif.

2. Dispositif selon la revendication 1, **caractérisé en ce que** les dispositifs d'amenée de fluide (14-29) sont conçus de façon telle que plusieurs tampons de fluide (13) sont expulsés les uns après les autres, de préférence selon des intervalles réguliers.

3. Dispositif selon l'une des revendications 1 à 2, **caractérisé en ce que** les dispositifs d'amenée de fluide (14-29) comportent des seconds dispositifs de réglage destinés à régler la pression (p₁) du gaz.

4. Dispositif selon la revendication 2, **caractérisé en ce que** les dispositifs d'amenée de fluide (14-29) comportent des troisièmes dispositifs de réglage (24) destinés à régler les écarts des tampons de fluide (13) selon lesquels ceux-ci sont expulsés.

5. Dispositif selon l'une des revendications 1 à 4, **caractérisé en ce que** les dispositifs d'amenée de fluide (14-29) comportent des soupapes (18-20) commandables, par l'intermédiaire desquelles la conduite (10) peut être reliée en alternance avec une source de fluide (17) et une source de gaz sous pression (16).

6. Dispositif selon l'une des revendications 1 à 5, **caractérisé en ce que** le segment proximal de la conduite (10) est relié, par l'intermédiaire d'une soupape (26) (de retenue) pouvant être traversée uniquement dans la direction de l'embouchure (11), à une source de fluide (17) qui se trouve sous une première pression (p₁) et est de plus reliée à une source de gaz sous pression (16) dont la pression (p₂) passe en alternance dans le temps au-dessous de et au-dessus de la première pression (p₁).
